# EUROPÄISCHE PATENTSCHRIFT

(11) **EP 3 695 826 B1**
(45) Veröffentlichungstag und Bekanntmachung des Hinweises auf die Patenterteilung: **29.10.2025**
(21) Anmeldenummer: 20159767.1
(22) Anmeldetag: 30.01.2018
(51) Int. Cl.: A61K 8/25, A61Q 17/04, A61K 8/02, A61K 8/06, A61K 8/37, A61K 8/39, A61K 8/44, A61K 8/46

(54) **VERWENDUNG VON GLASPERLEN AUS NATRON-KALK-BOROSILIKAT IN EINER KOSMETISCHEN ZUBEREITUNG ZUR REDUZIERUNG DER SANDANHAFTUNG AUF DER HAUT**
USE OF SODA-LIME-BOROSILICATE IN A COSMETIC COMPOSITION TO REDUCE ADHESION OF SAND ON THE SKIN
UTILISATION DES BILLES DE VERRE DE BOROSILICATE SODOCALCIQUE DANS UNE COMPOSITION COSMETIQUE POUR REDUIRE L'ADHERENCE DU SABLE SUR LA PEAU

(30) Priorität: 07.02.2017 DE 102017201869
(43) Veröffentlichungstag der Anmeldung: 19.08.2020
(62) Teilanmeldung aus: 18702662.0
(73) Patentinhaber: Beiersdorf AG, 22529 Hamburg (DE)
(72) Erfinder: Schlenker, David, 22607 Hamburg (DE); Heitmann, Birgit, 22885 Barsbüttel (DE); Bleckmann, Andreas, 22926 Ahrensburg (DE)
(74) Vertreter: Beiersdorf AG

(56) Entgegenhaltungen:
- FR-A1- 2 863 877
- US-A- 5 766 611
- US-A1- 2008 219 939
- "Suncare compositions with new cosmetic raw materials (2) ED - Darl Kuhn", IP.COM, IP.COM INC., WEST HENRIETTA, NY, US, 2 August 2010 (2010-08-02), XP013143636, ISSN: 1533-0001

## Beschreibung

Die vorliegende Erfindung betrifft die Verwendung von Glasperlen aus Natron-Kalk-Borosilikat in kosmetischen Zubereitungen.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Die Hautreinigung erfolgt in der Regel mit Hilfe von oberflächenaktiven Zubereitungen (Seifen, Tenside, seltener alkoholische Zubereitungen), die in Form von schaumbildenen Gelen oder Feststoffen (Seifenstücke) vorliegen und nach dem Auftragen auf die Haut mit Wasser wieder abgespült werden.

Hautpflegeprodukte, in der Regel Crémes, Salben oder Lotionen, dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Die Vielzahl an kommerziell erhältlichen Hautpflegeprodukten, zu denen auch die Sonnenschutzmittel zählen, darf jedoch nicht darüber hinwegtäuschen, dass diese Zubereitungen des Standes der Technik eine Reihe von Nachteilen aufweisen.

Kosmetische Hautpflegeprodukte, selbst solche auf Basis einer ethanolischen Lösung, enthalten üblicherweise Ölkomponenten und andere mehr oder weniger klebrige Inhaltsstoffe, die dazu führen, dass staubförmige, feinkörnige Partikel stärker auf der Haut . haften bleiben. Dieses Problem tritt besonders störend bei Sonnenschutzmitteln auf, die von Verbrauchern am Strand angewendet werden. Die Problematik der sogenannten Sandanhaftung bei Sonnenschutzmitteln ist wiederholt in der Literatur beschrieben worden. Das Problem tritt jedoch auch bei anderen kosmetischen Produkten und anderen Alltagssituationen auf, beispielsweise bei Tages- oder Nachcremes in Verbindung mit Hausstaub oder Pollen.

EP 1 506 772 A2 setzt Glas-Mikrokugeln (Glasperlen) in Sonnenschutzmittel ein, um deren Klebrigkeit zu verringern. Als Beispiele werden Natron-Kalk-Borosilikatkugeln genannt.

Es war daher die Aufgabe der vorliegenden Erfindung, die durch die kosmetische Zubereitung hervorgerufene Partikelanhaftung auf der Haut und insbesondere die von den Kosmetika (insbesondere Sonnenschutzmitteln) hervorgerufene Sandanhaftung auf der Haut zu reduzieren.

Eine nach dem Stand der Technik mögliche Lösung der Aufgabe besteht darin, sogenannte Puderrohstoffe aus bei Raumtemperatur festen Kunststoffpartikeln in die Zubereitungen einzuarbeiten. Diese bei Raumtemperatur festen Kunststoffpartikeln bestehen aus Polyethylen, Polypropylen, Polymethylmethacrylat oder Nylon. Dies ist auch die erfindungsgemäße Definition für diese Stoffe. Nachteilig an diesem Stand der Technik ist nun der Umstand, dass der Einsatz dieser Stoffe im Zusammenhang mit der sogenannten "Mikroplastik"-Diskussion, d.h. der Diskussion über den Einsatz von Kunststoffpartikeln in Kosmetika und deren Verbleib nach der kosmetischen Anwendung in der Umwelt, zunehmend als unerwünscht angesehen wird. Ob und ggf. in welchem Umfang diese Diskussion berechtigt ist, kann im Rahmen dieser Offenbarung dahingestellt bleiben. Tatsache ist jedoch, dass bei den Verbrauchern ein steigendes Interesse an kosmetischen Produkten besteht, welche diese Inhaltstoffe nicht mehr aufweisen.

Es war daher die Aufgabe der vorliegenden Erfindung, die durch die kosmetische Zubereitung hervorgerufene Partikelanhaftung auf der Haut und insbesondere die von den Kosmetika (insbesondere Sonnenschutzmitteln) hervorgerufene Sandanhaftung auf der Haut zu reduzieren, ohne dafür Raumtemperatur feste Kunststoffpartikel, d.h. Polyethylen, Polypropylen, Polymethylmethacrylat oder Nylon einzusetzen.

Überraschend gelöst werden diese Aufgaben durch eine Verwendung gemäß Anspruch 1.

Dabei ist es erfindungsgemäß bevorzugt, wenn die Zubereitung frei ist von bei Raumtemperatur festen Kunststoffpartikeln.

Zwar kennt der Stand der Technik Glasperlen für kosmetische Zubereitungen, beispielsweise aus Alkali-Kalk-Glas (z.B. von GP Cosmetics) oder Magnesiumsilikat (z.B. Covabead Crystal von Sensient Cosmetic Technologies). Diese haben jedoch den Nachteil, bei Emulsionen, d.h. der am weitesten verbreiteten kosmetischen Zubereitungsform zu Verfärbungen der der Zubereitung zu führen. Stabilitätsprobleme gibt es auch mit auf Silica-Basis bestehenden verkapselten UV-Filtern (z.B. Eusolex UV-Pearls von Merck). Es war jedoch die Aufgabe der vorliegenden Erfindung, stabile (insbesondere farblich stabile) Zubereitungen, insbesondere Emulsionen (und hier O/W-Emulsionen) zu entwickeln.

Es ist erfindungsgemäß vorteilhaft, wenn die erfindungsgemäßen Glasperlen einen Partikeldurchmesser von 5 bis 120 µm aufweisen.

Die erfindungsgemäße Teilchengröße (Partikeldurchmesser) der Glasperlen kann dabei entsprechend den üblichen Normen beispielsweise durch Sieben bestimmt werden. Dabei ist dem Fachmann natürlich bekannt, dass die Teilchengröße (Korngröße) immer einer gewissen Größenverteilung unterliegt. Die Größenangabe bedeutet daher, dass keine größeren Glasperlen als jene mit einem Partikeldurchmesser von 120 µm eingesetzt werden und es unkritisch ist, wenn geringe Mengen an kleineren Partikeln als Verunreinigungen (beispielsweise entstanden durch "Glasbruch") in der Zubereitung enthalten sind.

Es ist erfindungsgemäß vorteilhaft, wenn die erfindungsgemäßen Glasperlen hohl sind.

Erfindungsgemäß vorteilhafte Ausführungsformen der vorliegenden Erfindung sind dadurch gekennzeichnet, dass die Dichte der Glasperlen 0,1 bis 1,0 g/Kubikzentimeter beträgt. Dabei ist eine Dichte von 0,13 bis 0,63 erfindungsgemäß bevorzugt.

Darüber hinaus ist es erfindungsgemäß von Vorteil, wenn die Glasperlen eine Ölabsorption von 0,2 bis 0,6 Gramm Öl pro Kubikzentimeter Glasperlen, gemessen nach ASTM D281-95 aufweisen. Dabei ist eine Ölabsorption von 0,3 bis 0,5 Gramm Öl pro Kubikzentimeter Glasperlen, gemessen nach ASTM D281-95, erfindungsgemäß bevorzugt.

Vorteilhaft im Sinne der vorliegenden Erfindung ist es, wenn die Glasperlen einen pH-Wert von 9 bis 10 aufweisen, gemessen nach ASTM D3100-1982.

Es ist erfindungsgemäß von Vorteil, wenn die Glasperlen eine Erweichungstemperatur von 550 bis 650 °C aufweisen. Dabei wird die Erweichung nach 2 Stunden Lagerung bei der entsprechenden Temperatur ermittelt. Erfindungsgemäß bevorzugt ist eine Erweichungstemperatur von 590 bis 610 °C, wobei sie idealerweise bei 600 °C liegt.

Es ist erfindungsgemäß von Vorteil, wenn die Zubereitung die Glasperlen in einer Menge von 0,1 bis 10 Gewichts-%, bezogen auf das Gesamtgewicht der Zubereitung, enthält. Dabei ist ein Gehalt von 1 bis 6 Gewichts-%, bezogen auf das Gesamtgewicht der Zubereitung, erfindungsgemäß bevorzugt.

Erfindungsgemäß vorteilhafte Ausführungsformen der vorliegenden Erfindung sind dadurch gekennzeichnet, dass die Ölphase der Zubereitung eine oder mehrere Ölkomponenten gewählt aus der Gruppe der Verbindungen Diisopropyladipat, Capryl/Caprinsäuretriglycerid (INCI Caprylic/Capric Triglycerides), Isopropylpalmitat, Dimethicon, Cyclomethicon, Octyldodecanol, Ethylhexylcocoat, Myristylmyristat, Hydrierte Cocosglyceride (INCI Hydrogenated Coco-Glycerides), Dicaprylylcarbonat, C18-38 Alkylhydroxystearorylstearat (INCI C18-38 Alkyl Hydroxystearoyl Stearate), Di-n-butyladipat, Butylenglycoldicaprylat/Dicaprat (INCI Butylene Glycol Dicaprylate/Dicaprate), C12-15 Alkylbenzoat (INCI C12-15 Alkyl Benzoate), 2-Phenylethylbenzoat (INCI Phenethyl Benzoate), Di C12-13 Alkyltatrat (INCI Di-C12-13 Alkyl Tatrate), Butylenglycolcocoat (INCI: Butylene Glycol Cocoate), Dicaprylylether, Isodecylneopentanoat, Tridecyltrimelliat, Isopropylstearat, C12.13 Alkyllactat, 2-Propylheptyloctanoat, Isopropyllaurylsarcosinat, enthält.

Die erfindungsgemäß bevorzugten Ölkomponenten sind dabei Diisopropyladipat, Capryl/Caprinsäuretriglycerid (INCI Caprylic/Capric Triglycerides), Octyldodecanol, Ethylhexylcocoat, Myristylmyristat, Hydrierte Cocosglyceride (INCI Hydrogenated Coco-Glycerides), Di-n-butyladipat, Butylenglycoldicaprylat/Dicaprat (INCI Butylene Glycol Dicaprylate/Dicaprate), C12-15 Alkylbenzoat (INCI C12-15 Alkyl Benzoate), 2-Phenylethylbenzoat (INCI Phenethyl Benzoate).

Es ist erfindungsgemäß von Vorteil, wenn die Gesamtmenge dieser erfindungsgemäß vorteilhaften Ölkomponenten von 1 bis Gewichts 20 %, bezogen auf das Gesamtgewicht der Zubereitung beträgt, wobei der bevorzugte Mengenbereich von 2 bis Gewichts 15 %, bezogen auf das Gesamtgewicht der Zubereitung darstellt.

Es ist erfindungsgemäß vorteilhaft, wenn die erfindungsgemäße Zubereitung einen oder mehrere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenme-thyl)benzolsulfonsäuresalze; 2-Methyl-5-(2-oxo-3-bornylidenmethyl)sulfonsäuresalze; 2-(2H-benzotriazol-2-yl)-4-methyl-6-[2-methyl-3-[1,3,3,3-tetramethyl-1-[(trimethylsilyl)oxy]disiloxa-nyl]propyl]-phenol; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydiben-zoylmethan; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-meth-oxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin; 4-Dicyanomethylen-2,6-dimethyl-1,4-dihydropyridin-N-(ethyloxysulfatestersalze), Titandioxid; Zinkoxid.

Darüber hinaus ist der Einsatz von 4-(tert.-Butyl)-4'-methoxydibenzoylmethan, ; 2-(4'-Diethylamino-2'-hydoxybenzoyl)-benzoesäurehexylester (INCI: Diethylamino hydroxybenzoyl hexyl benzoate), 2-Phenylbenzimidazol-5-sulfonsäuresalzen, Ethylhexylsalicylat; Dioctylbutylamidotriazon (INCI: Diethylhexyl-Butamidotriazone), 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone) und 2,4-Bis-{[4-(2-ethyl-hexyloxy)-2-hydroxy]-phenyl}-6-(4-methoxyphenyl)-1,3,5-triazin erfindungsgemäß besonders bevorzugt, wobei diese UV-Filter einzeln oder als Gemisch eingesetzt werden können.

Es ist vorteilhaft im Sinne der vorliegenden Erfindung, wenn die Zubereitung ein oder mehrere Alkohole gewählt aus der Gruppe der Verbindungen Ethanol, Glycerin, Ethylhexylglycerin, Phenoxyethanol, Polyglyceryl-2 Caprate, Propylenglycol, Butylenglycol, 2-Methylpropan-1,3-diol, 1,2-Pentandiol, 1,2-Hexandiol, 1,2-Octandiol, 1,2-Decandiol enthält. Diese Emulgatoren können in einer Gesamtkonzentration von 0,2 bis 80 Gewichts-%, bezogen auf das Gesamtgewicht der Zubereitung eingesetzt werden.

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Isoflavonoide, Flavonoide, Kreatin, Kreatinin, Taurin, β-Alanin, Panthenol, Polydocanol, Tocopherylacetat, Dihydroxyaceton, Glycerylglycose, (2-Hydroxyethyl)harnstoff, Vitamin E bzw. Vitamin E Acetat, Hyaluronsäure und/oder deren Natriumsalze, Menthol, Glycyrrhetinsäure und/oder Licochalcon A enthält.

Nicht zuletzt ist es erfindungsgemäß von Vorteil, wenn die Zubereitung ein oder mehrere Polymere gewählt aus der Gruppe der Verbindungen Xanthamgummi, Tapiokastärke, Hydroxyethylcellulose, Carbomer, Acrylat/C10-30 Alkylacrylat, Vinylpyrrolidon/Hexadecencopolymer, enthält.

Darüber hinaus kann die kosmetische Zubereitung wie ein für solche Fälle übliches Kosmetikum zusammengesetzt sein und die entsprechenden, bekannten Inhaltsstoffe enthalten.

### Vergleichsversuch

Es wurden die folgenden Rezepturen hergestellt und die Sandanheftung mit Hilfe der folgenden Methode bestimmt.

| inci | **sweat 249** | **_sweat_260** | **_sweat_261** |
|---|---|---|---|
| Glasperlen größter Durchmesser ca. 150 µm (z.B. 3M Glass Bubbles K15) | 0 | 5 | 0 |
| Glasperlen größter Durchmesser ca. 30 µm (z.B. 3M GLASS BUBBLES IM30K) | 0 | 0 | 5 |
| Tocopheryl Acetate | 0,11 | 0,11 | 0,11 |
| Menthol | 0,05 | 0,05 | 0,05 |
| C12-15 Alkyl Benzoate | 5 | 5 | 5 |
| Diisopropyl Adipate | 3 | 3 | 3 |
| Butylene Glycol Dicaprylate/Dicaprate | 4 | 4 | 4 |
| Polyglyceryl-3 Methylglucose Distearate | 0,2 | 0,2 | 0,2 |
| Silica Dimethyl Silylate | 1 | 1 | 1 |
| Tapioca Starch + Aqua | 4 | 4 | 4 |
| Parfum | 0,4 | 0,4 | 0,4 |
| Glycerin + Aqua | 1 | 1 | 1 |
| Aqua + Sodium Hydroxide | 0,623 | 0,623 | 0,623 |
| Phenoxyethanol | 0,5 | 0,5 | 0,5 |
| Hydroxyethylcellulose | 0,1 | 0,1 | 0,1 |
| Carbomer | 0,125 | 0,125 | 0,125 |
| Acrylates/C10-30 Alkyl Acrylate Crosspolymer | 0,2 | 0,2 | 0,2 |
| Xanthan Gum | 0,2 | 0,2 | 0,2 |
| Aqua | ad 100 | ad 100 | ad 100 |
| Alcohol Denat. + Aqua | 10 | 10 | 10 |
| Aqua + Trisodium EDTA | 1 | 1 | 1 |
| Ethylhexyl Salicylate | 5 | 5 | 5 |
| Bis-Ethylhexyloxyphenol Methoxyphenyl Triazine | 4 | 4 | 4 |
| Ethylhexyl Triazone | 3 | 3 | 3 |
| Butyl Methoxydibenzoylmethane | 4,75 | 4,75 | 4,75 |
| Phenylbenzimidazole Sulfonic Acid | 1 | 1 | 1 |

### In-vitro Sandanhaftung

50 mg der Test-Emulsion wurden auf PMMA Schönberg Platten (5,0 x 5,0 cm) aufgetragen und gleichmäßig mit einem Fingerling auf der Platte verteilt. Anschließend wird die aufgetragene Beispielrezeptur für 15 min bei Raumtemperatur getrocknet. Danach wurde das Gewicht der getrockneten Platten mit einer Analysenwaage ermittelt. Anschließend wurden die Platten mit feinem Seesand (1.07711.1000 Seesand reinst, der Firma Merck KGaA) im Überschuss übergossen. Durch einmaliges Rutschen der Platten auf einer dafür vorgesehenen Rutschvorrichtung (siehe unten) wurde lose anhaftender Sand mit reproduzierbarer, einheitlicher Kraft entfernt.

Der daraufhin auf der Platte zurückbleibende anhaftende Sand wurde durch Auswiegen ermittelt. Die Sandanhaftung kann mit folgender Gleichung ermittelt werden:
Die Rutschvorrichtung ist eine in Form eines Dreiecks aufgebaute Konstruktion, bei der die Breite der Rutsche 5 cm beträgt. Die Rutschvorrichtung ist eine in Form eines rechtwinkligen Dreiecks aufgebaute Konstruktion, bei der die Breite der Rutsche 5 cm beträgt. Die Ankathete des rechtwinkeligen Dreiecks (= Standfläche der Rutsche) hat eine Länge von 13,5 cm, die Gegenkathete (= Fallhöhe) hat eine Länge von 49 cm. Die Hypotenuse, auf welcher der Rutschvorgang stattfindet, und die Standfläche schließen einen Winkel von 275 ° ein.

Die Versuche wurden je Rezeptur 10x wiederholt und der entsprechende Mittelwert gebildet.

Einzeldaten Sandanhaftung:

| **Muster** | **w Platte in q** | **w Platte+Sand in q** | **w Sand absolut in q** | **Mittelwert** | **Standardabweichung** |
|---|---|---|---|---|---|
| SW EAT:249 a-15 | 7.292 | 7.35 | 0.058 | | |
| SW EAT:249 b-15 | 7.297 | 7.423 | 0.126 | | |
| SWAET:249 c-15 | 7.299 | 7.429 | 0.13 | | |
| SWAET:249 d-15 | 7.27 | 7.376 | 0.106 | | |
| SWEAT:249 e-15 | 7.311 | 7.395 | 0.084 | | |
| SW EAT:249 f-15 | 7.279 | 7.385 | 0.106 | | |
| SW EAT:249 g-15 | 7.169 | 7.287 | 0.118 | | |
| SWEAT:249 h-15 | 7.301 | 7.35 | 0.049 | | |
| SWEAT:249 i-15 | 7.305 | 7.434 | 0.129 | | |
| SW EAT:249 j-15 | 7.29 | 7.396 | 0.106 | 0.101 | 0.029 |
| | | | | | |
| SWEAT:260 a-15 | 7.292 | 7.294 | 0.002 | | |
| SWEAT:260 b-15 | 7.311 | 7.314 | 0.003 | | |
| SWAET:260 c-15 | 7.216 | 7.218 | 0.002 | | |
| SWAET:260 d-15 | 7.152 | 7.154 | 0.002 | | |
| SWEAT:260 e-15 | 7.106 | 7.11 | 0.004 | | |
| SWEAT:260 f-15 | 7.285 | 7.287 | 0.002 | | |
| SWEAT:260 g-15 | 7.172 | 7.179 | 0.007 | | |
| SW EAT:260 h-15 | 7.166 | 7.17 | 0.004 | | |
| SWEAT:260 i-15 | 7.199 | 7.207 | 0.008 | | |
| SWEAT:260 j-15 | 7.323 | 7.329 | 0.006 | 0.004 | 0.002 |
| | | | | | |
| SWEAT:261 a-15 | 7.264 | 7.301 | 0.037 | | |
| SWEAT:261 b-15 | 7.249 | 7.252 | 0.003 | | |
| SWAET:261 c-15 | 7.313 | 7.322 | 0.009 | | 0 |
| SWAET:261 d-15 | 7.323 | 7.327 | 0.004 | | |
| SWEAT:261 e-15 | 7.298 | 7.318 | 0.02 | | |
| SWEAT:261 f-15 | 7.307 | 7.317 | 0.01 | | |
| SWEAT:261 g-15 | 7.325 | 7.332 | 0.007 | | |
| SWEAT:261 h-15 | 7.291 | 7.299 | 0.008 | | |
| SWEAT:261 i-15 | 7.291 | 7.295 | 0.004 | | |
| SWEAT:261 j-15 | 7.325 | 7.331 | 0.006 | 0.011 | 0.010 |

| **Muster** | | **anhaftender Sand in mg/cm² (15 Minuten)** | | **Standardabweichung** | |
|---|---|---|---|---|---|
| SWEAT:249 N-free | | 4,0 | | 1,16 | |
| SWEAT:260 N-free + 5 % 3M Glass Bubbles K15 | | 0,2 | | 0,08 | |
| SWEAT:261 N-free + 5 % 3M Glass Bubbles IM30K | | 0,4 | | 0,4 | |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiele** | **1** | **2** | **3** | **4** | **5** | **6** | **7** | **8** | **9** | **10** |
|---|---|---|---|---|---|---|---|---|---|---|
| **INCI** | **m[%]** | **m[%]** | **m[%]** | **m[%]** | **m[%]** | **m[%]** | **m[%]** | **m[%]** | **m[%]** | **m[%]** |
| **Glasperlen größter Durchmesser ca. 30 µm (z.B. 3M GLASS BUBBLES IM30K)** | **2,5** | **5** | | | **2** | **6** | | | **3** | |
| **Glasperlen größter Durchmesser ca. 150 µm (z.B. 3M Glass Bubbles K15)** | | | **2,5** | **5** | | | **2** | **6** | | **3** |
| VP/Hexadecene Copolymer | 0,5 | 1 | | 0,5 | | 1 | | 0,5 | | |
| Tocopherylacetate | 0,06 | 0,1 | 0,01 | 0,15 | 0,06 | 0,5 | 0,1 | 0,06 | 0,15 | 0,1 |
| Panthenol | 1 | 1,4 | | 0,5 | 0,5 | | 1 | 1 | | 0,5 |
| Ethylhexylglycerin | 0,3 | 0,5 | 0,25 | | | | 0,3 | | | 0,25 |
| 1,2-Hexandiole | | | | 0,4 | | 1 | | | 1 | |
| Methylpropanediole | | | | | 0,2 | | | 0,2 | | |
| C12-15 Alkylbenzoate | 4,5 | 6 | 3 | | | 6 | 7 | | 7,5 | |
| Caprylic/Capric Triglycerides | | 4 | | | 2 | | | | 3 | |
| Isopropyl Palmitate | | | 4 | | | | | 3 | | 3 |
| Dimethicone | | | | 2 | 1 | | | 0,5 | | |
| Octyldodecanol | | | | 2 | | | | | 2 | |
| Ethylhexyl Cocoate | | | | | | 1 | | | 2 | 3 |
| Myristyl Myristate | 2 | 1 | 1,5 | | | 1 | | | | |
| Cetearyl Alcohol | | | | 1 | | | 1,5 | | | 1 |
| Hydrogenated Coco-Glycerides | | | | | 5 | | | 2 | | 1 |
| Butylene Glycol Dicaprylate/Dicaprate | 1 | | 3 | 1,5 | | | 2 | | | |
| Dicaprylyl Carbonate | 1 | | | 3 | | 3 | | | 1,5 | |
| C18-38 Alkyl Hydroxystearoyl Stearate | | | | | 0,5 | | | | | |
| Polyglyceryl-3 Methylglucose Distearate | | | | 0,15 | | | | | 0,15 | |
| Glyceryl Stearate Citrate | 2 | 3 | | | | 2 | | 2 | | |
| Glyceryl Stearate | | | 1 | | 1 | | | | | |
| Sodium Stearoyl Glutamate | | | | | | | 0,5 | | | |
| Glyceryl Stearate SE | | | | | | | 1 | | | 1 |
| Sodium Cetearal Sulfate | | | 0,15 | | 0,15 | | | | | 0,15 |
| Silica dimethyl Silylate | | 0,5 | 0,5 | | 0,3 | 1 | 0,5 | | 1 | |
| Tapioca starch | | | | | | | 3 | 3 | | |
| Parfum | 0,4 | 0,3 | 0,2 | 0,4 | | 0,4 | 0,5 | | 0,3 | 0,2 |
| Glycerin | 6 | 8 | 5 | 1 | 1 | 5 | 8 | 6 | 5 | 1 |
| Citric Acid | 0,07 | 0,09 | | | | 0,01 | | 0,008 | | |
| Sodium Citrate | 0,16 | 0,15 | | | | 0,16 | | 0,17 | | |
| Sodium Hydroxide | 0,05 | 0,08 | 0,07 | 0,08 | 0,05 | 0,03 | | 0,07 | 0,06 | 0,03 |
| Phenoxyethanol | 0,3 | 0,5 | 0,3 | 0,4 | 0,5 | 0,2 | | 0,5 | 0,3 | 0,4 |
| Methylparaben | 0,2 | 0,3 | 0,2 | 0,2 | 0,3 | | | 0,4 | 0,2 | 0,3 |
| Ethylparaben | 0,2 | 0,3 | 0,2 | 0,3 | | | | 0,2 | 0,3 | |
| Stearyl Alcohol | 0,8 | 0,8 | | | 1 | | 0,8 | 1,5 | | |
| Acrylates/C10-30Alkyl Acrylates Crosspolymer | 0,1 | 0,15 | 0,05 | | 0,2 | 0,1 | | 0,05 | 0,15 | 0,2 |
| Carbomer | | | | | 0,15 | | | | | |
| Hydroxyethylcellulose | | | | | 0,1 | | | | | |
| Xanthan Gum | 0,3 | 0,2 | 0,5 | 0,4 | | 0,3 | 0,4 | 0,2 | 0,3 | 0,3 |
| Cetyl Alcohol | 0,5 | 1 | | | | | | | 2 | |
| Alcohol Denat | 4 | 6 | 4 | 8 | 10 | 4 | 6 | 6 | 8 | 10 |
| Trisodium EDTA | 1 | 0,5 | 1 | 1 | 1 | 0,5 | 0,5 | 1 | 1 | 1 |
| Homosalate | 9 | | 9 | 9 | 9,5 | 9 | 9 | 9 | 9 | 9 |
| Octocrylene | 9 | 9 | | | 9,5 | | | 8 | 8 | |
| Ethylhexyl Salicylate | 4,5 | | 4,75 | 4,75 | 4,75 | 4,75 | 4,75 | 4,75 | 4,75 | 4,75 |
| Butyl Methoxydibebzoylmethane | 4,5 | 4,5 | | 4,75 | 4,75 | 4,75 | 4,5 | 4,5 | 4,75 | 4,75 |
| Titanium dioxide | 3 | 6 | | | | | 1 | | 3,5 | |
| Bis-Ethylhexyloxyphenol Methoxyphenyl Triazine | | 3 | 4 | 4 | 3,5 | 4 | 4 | 3,5 | 3 | 4 |
| Ethylhexyl Triazone | | | 3 | 3 | | 3 | 3 | | | 3 |
| Diethylamino Hydroxybenzoyl Hexvlbenzoate | | | 8 | | | | | | | |
| Polysilicone-15 | | 1 | | | | | | | | |
| Phenylbenzimidazole Sulfonic Acid | | | 1 | 1 | 1 | 1 | | 1,5 | 1,5 | 1 |
| Aqua | ad 100 | ad 100 | ad 100 | ad 100 | ad 100 | ad 100 | ad 100 | ad 100 | ad 100 | ad 100 |

## Patentansprüche

1. Verwendung von Glasperlen aus Natron-Kalk-Borosilikat in einer kosmetischen Zubereitung enthaltend mindestens eine Ölphase, wobei die Zubereitung in Form einer O/W-Emulsion vorliegt, die mit einem oder mehreren Emulgatoren emulgiert ist, die gewählt werden aus der Gruppe der Verbindungen Glycerylstearatcitrat, Glycerylstearat (selbstemulgierend), Polyglyceryl-3-methylglycosedistearat, Natriumstearoylglutamat, Natriumcetearylsulfat, Kaliumcetylphosphat, Cetearylsulfosuccinat, Polyglyceryl-10 Stearat, Polyglyceryl-10 Laurat, Polyglyceryl-10 Caprat, wobei die Zubereitung frei ist von 2-Ethylhexyl-2-cyano-3,3-diphenylacrylat, 2-Hydroxy-4-methoxybenzophenon, 4-Methoxyzimtsäure(2-ethylhexyl)ester, 4-Methoxyzimtsäureisoamylester und 3-(4-Methylbenzyliden)campher, zur Reduzierung der Sandanhaftung auf der Haut.

2. Verwendung nach Anspruch 1, **dadurch gekennzeichnet, dass** die Zubereitung frei ist von bei Raumtemperatur festen Kunststoffpartikeln.

3. Verwendung nach einem der vorhergehenden Ansprüche, **dadurch gekennzeichnet, dass** die Glasperlen einen Partikeldurchmesser von 5 bis 120 µm aufweisen.

4. Verwendung nach einem der vorhergehenden Ansprüche, **dadurch gekennzeichnet, dass** die Glasperlen hohl sind.

5. Verwendung nach einem der vorhergehenden Ansprüche, **dadurch gekennzeichnet, dass** die Dichte der Glasperlen 0,1 bis 1,0 g/Kubikzentimeter beträgt.

6. Verwendung nach einem der vorhergehenden Ansprüche, **dadurch gekennzeichnet, dass** die Glasperlen eine Ölabsorption von 0,2 bis 0,6 Gramm Öl pro Kubikzentimeter Glasperlen, gemessen nach ASTM D281-95

7. Verwendung nach einem der vorhergehenden Ansprüche, **dadurch gekennzeichnet, dass** die Glasperlen einen pH-Wert von 9 bis 10 aufweisen, gemessen nach ASTM D3100-1982.

8. Verwendung nach einem der vorhergehenden Ansprüche, **dadurch gekennzeichnet, dass** die Glasperlen eine Erweichungstemperatur von 550 bis 650 °C aufweisen.

9. Verwendung nach einem der vorhergehenden Ansprüche, **dadurch gekennzeichnet, dass** die Zubereitung die Glasperlen in einer Menge von 0,1 bis 10 Gewichts-%, bezogen auf das Gesamtgewicht der Zubereitung, enthält.

10. Verwendung nach einem der vorhergehenden Ansprüche, **dadurch gekennzeichnet, dass** die Ölphase der Zubereitung eine oder mehrere Ölkomponenten enthält, die gewählt werden aus der Gruppe der Verbindungen Diisopropyladipat, Capryl/Caprinsäuretriglycerid (INCI Caprylic/Capric Triglycerides), Isopropylpalmitat, Dimethicon, Cyclomethicon, Octyldodecanol, Ethylhexylcocoat, Myristylmyristat, Hydrierte Cocosglyceride (INCI Hydrogenated Coco-Glycerides), Dicaprylylcarbonat, C18-38 Alkylhydroxystearorylstearat (INCI C18-38 Alkyl Hydroxystearoyl Stearate), Di-n-butyladipat, Butylenglycoldicaprylat/Dicaprat (INCI Butylene Glycol Dicaprylate/Dicaprate), C12-15 Alkylbenzoat (INCI C12-15 Alkyl Benzoate), 2-Phenylethylbenzoat (INCI Phenethyl Benzoate), Di C12-13 Alkyltatrat (INCI Di-C12-13 Alkyl Tatrate), Butylenglycolcocoat (INCI: Butylene Glycol Cocoate), Dicaprylylether, Isodecylneopentanoat, Tridecyltrimelliat, Isopropylstearat, C12.13 Alkyllactat, 2-Propylheptyloctanoat, Isopropyllaurylsarcosinat.

11. Verwendung nach einem der vorhergehenden Ansprüche, **dadurch gekennzeichnet, dass** die Zubereitung einen oder mehrere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2-(2H-benzotriazol-2-yl)-4-methyl-6-[2-methyl-3-(1,3,3,3-tetramethyl-1-[(trimethylsilyl)oxy]disiloxanyl]propyl]-phenol; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoäsäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-([4-(2-ethyl-hexyloxy)-2-hydroxy]-phenyl}-6-(4-methoxyphenyl)-1,3,5-triazin; 4-Dicyanomethylen-2,6-dimethyl-1,4-dihydropyridin-N-(ethyloxysulfatestersalze), Titandioxid; Zinkoxid.

12. Verwendung nach einem der vorhergehenden Ansprüche, **dadurch gekennzeichnet, dass** die Zubereitung ein oder mehrere Alkohole gewählt aus der Gruppe der Verbindungen Ethanol, Glycerin, Ethylhexylglycerin, Phenoxyethanol, Polyglyceryl-2 Caprate, Propylenglycol, Butylenglycol, 2-Methylpropan-1,3-diol, 1,2-Pentandiol, 1,2-Hexandiol, 1,2-Octandiol, 1,2-Decandiol enthält.

13. Verwendung nach einem der vorhergehenden Ansprüche, dadurch gekennz4eichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Isoflavonoide, Flavonoide, Kreatin, Kreatinin, Taurin, β-Alanin, Panthenol, Polydocanol, Tocopherylacetat, Dihydroxyaceton, Glycerylglycose, (2-Hydroxyethyl)harnstoff, Vitamin E bzw. Vitamin E Acetat, Hyaluronsäure und/oder deren Natriumsalze, Menthol, Glycyrrhetinsäure und/oder Licochalcon A enthält.

14. Verwendung nach einem der vorhergehenden Ansprüche, **dadurch gekennzeichnet, dass** die Zubereitung ein oder mehrere Polymere gewählt aus der Gruppe der Verbindungen Xanthamgummi, Tapiokastärke, Hydroxyethylcellulose, Carbomer, Acrylat/C10-30 Alkylacrylat, Vinylpyrrolidon/Hexadecencopolymer, enthält.

## Claims

1. Use of soda-lime-borosilicate glass beads in a cosmetic preparation comprising at least one oil phase, wherein the preparation is in the form of an O/W emulsion, which has been emulsified with one or more emulsifiers selected from the group of compounds comprising glyceryl stearate citrate, glyceryl stearate (self-emulsifying), polyglyceryl-3 methylglycose distearate, sodium stearoyl glutamate, sodium cetearyl sulfate, potassium cetyl phosphate, cetearyl sulfosuccinate, polyglyceryl-10 stearate, polyglyceryl-10 laurate, polyglyceryl-10 caprate, wherein the preparation is free of 2-ethylhexyl 2-cyano-3,3-diphenylacrylate, 2-hydroxy-4-methoxybenzophenone, 2-ethyl hexyl 4-methoxycinnamate, isoamyl 4-methoxycinnamate and 3-(4-methylbenzylidene)camphor, for reducing the adhesion of sand to the skin.

2. Use according to Claim 1, **characterized in that** the preparation is free of plastic particles solid at room temperature.

3. Use according to either of the preceding claims, **characterized in that** the glass beads have a particle diameter of 5 to 120 µm.

4. Use according to any of the preceding claims, **characterized in that** the glass beads are hollow.

5. Use according to any of the preceding claims, **characterized in that** the density of the glass beads is 0.1 to 1.0 g/cubic centimetre.

6. Use according to any of the preceding claims, **characterized in that** the glass beads have an oil absorption of 0.2 to 0.6 grams of oil per cubic centimetre of glass beads, measured in accordance with ASTM D281-95.

7. Use according to any of the preceding claims, **characterized in that** the glass beads have a pH of 9 to 10, measured in accordance with ASTM D3100-1982.

8. Use according to any of the preceding claims, **characterized in that** the glass beads have a softening temperature of 550 to 650°C.

9. Use according to any of the preceding claims, **characterized in that** the preparation comprises the glass beads in an amount of 0.1 to 10% by weight, based on the total weight of the preparation.

10. Use according to any of the preceding claims, **characterized in that** the oil phase of the preparation comprises one or more oil components selected from the group of compounds comprising diisopropyl adipate, caprylic/capric triglyceride (INCI Caprylic/Capric Triglycerides), isopropyl palmitate, dimethicone, cyclomethicone, octyldodecanol, ethylhexyl cocoate, myristyl myristate, hydrogenated coco-glycerides (INCI Hydrogenated Coco-Glycerides), dicaprylyl carbonate, C18-38 alkyl hydroxystearoryl stearate (INCI C18-38 Alkyl Hydroxystearoyl Stearate), di-n-butyl adipate, butylene glycol dicaprylate/dicaprate (INCI Butylene Glycol Dicaprylate/Dicaprate), C12-15 alkyl benzoate (INCI C12-15 Alkyl Benzoate), 2-phenylethyl benzoate (INCI Phenethyl Benzoate), di C12-13 alkyl tartrate (INCI Di-C12-13 Alkyl Tartrate), butylene glycol cocoate (INCI: Butylene Glycol Cocoate), dicaprylyl ether, isodecyl neopentanoate, tridecyl trimellitate, isopropyl stearate, C12.13 alkyl lactate, 2-propylheptyl octanoate, isopropyl lauryl sarcosinate.

11. Use according to any of the preceding claims, **characterized in that** the preparation comprises one or more UV filters selected from the group of compounds comprising phenylene-1,4-bis-(2-benzimidazyl)-3,3'-5,5'-tetrasulfonic acid salts; 2-phenylbenzimidazole-5-sulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2-(2H-benzotriazol-2-yl)-4-methyl-6-[2-methyl-3-[1,3,3,3-tetramethyl-1-[(trimethylsilyl)oxy]disiloxanyl]propyl]phenol; 3-benzylidenecamphor; ethylhexyl salicylate; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; 2-hydroxy-4-methoxy-4'-methylbenzophenone; 2,2'-dihydroxy-4-methoxybenzophenone; hexyl 2-(4'-diethylamino-2'-hydroxybenzoyl)benzoate; 4-(tert-butyl)-4'-methoxydibenzoylmethane; homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxysiloxane / dimethylsiloxane - copolymer; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4-bis{[4-(2-ethylhexyloxy)-2-hydroxy]phenyl}-6-(4-methoxyphenyl)-1,3,5-triazine; 4-dicyanomethylene-2,6-dimethyl-1,4-dihydropyridine N-(ethyloxysulfate ester salts), titanium dioxide; zinc oxide.

12. Use according to any of the preceding claims, **characterized in that** the preparation comprises one or more alcohols selected from the group of compounds comprising ethanol, glycerol, ethylhexylglycerin, phenoxyethanol, polyglyceryl-2 caprate, propylene glycol, butylene glycol, 2-methylpropane-1,3-diol, 1,2-pentanediol, 1,2-hexanediol, 1,2-octanediol, 1,2-decanediol.

13. Use according to any of the preceding claims, **characterized in that** the preparation comprises one or more compounds selected from the group of compounds comprising alpha-lipoic acid, folic acid, phytoene, D-biotin, coenzyme Q10, alpha-glucosylrutin, carnitine, carnosine, isoflavonoids, flavonoids, creatine, creatinine, taurine, β-alanine, panthenol, polidocanol, tocopheryl acetate, dihydroxyacetone, glycerylglucose, (2-hydroxyethyl)urea, vitamin E and vitamin E acetate, hyaluronic acid and/or sodium salts thereof, menthol, gylcyrrhetic acid and/or licochalcone A.

14. Use according to any of the preceding claims, **characterized in that** the preparation comprises one or more polymers selected from the group of compounds comprising xanthan gum, tapioca starch, hydroxyethylcellulose, carbomer, acrylate/C10-30 alkyl acrylate, vinylpyrrolidone/hexadecene copolymer.

## Revendications

1. Utilisation de perles de verre de borosilicate sodo-calcique dans une préparation cosmétique contenant au moins une phase huileuse, la préparation se présentant sous forme d'une émulsion H/E qui est émulsionnée avec un ou plusieurs émulsifiants qui sont choisis dans le groupe des composés citrate-stéarate de glycéryle, stéarate de glycéryle (autoémulsifiant), distéarate de polyglycéryl-3-méthylglucose, stéaroylglutamate de sodium, cétéarylsulfate de sodium, cétylphosphate de potassium, cétéarylsulfosuccinate, stéarate de polyglycéryle-10, laurate de polyglycéryle-10, caprate de polyglycéryle-10, la préparation étant exempte de 2-cyano-3,3-diphénylacrylate de 2-éthylhexyle, 2-hydroxy-4-méthoxybenzophénone, 4-méthoxycinnamate de 2-éthylhexyle, 4-méthoxycinnamate d'isoamyle et 3-(4-méthylbenzylidène)camphre, pour la réduction de l'adhérence du sable à la peau.

2. Utilisation selon la revendication 1, **caractérisée en ce que** la préparation est exempte de particules de matière plastique solides à la température ambiante.

3. Utilisation selon l'une quelconque des revendications précédentes, **caractérisée en ce que** les perles de verre présentent un diamètre de particule de 5 à 120 µm.

4. Utilisation selon l'une quelconque des revendications précédentes, **caractérisée en ce que** les perles de verre sont creuses.

5. Utilisation selon l'une quelconque des revendications précédentes, **caractérisée en ce que** la densité des perles de verre vaut de 0,1 à 1,0 g/centimètre cube.

6. Utilisation selon l'une quelconque des revendications précédentes, **caractérisée en ce que** les perles de verre présentent une absorption d'huile de 0,2 à 0,6 gramme d'huile par centimètre cube de billes de verre, mesurée selon ASTM D281-95.

7. Utilisation selon l'une quelconque des revendications précédentes, **caractérisée en ce que** les perles de verre ont un pH de 9 à 10, mesuré selon ASTM D3100-1982.

8. Utilisation selon l'une quelconque des revendications précédentes, **caractérisée en ce que** les perles de verre présentent une température de ramollissement de 550 à 650 °C.

9. Utilisation selon l'une quelconque des revendications précédentes, **caractérisé en ce que** la préparation contient les perles de verre en une quantité de 0,1 à 10 % en poids par rapport au poids total de la préparation.

10. Utilisation selon l'une quelconque des revendications précédentes, **caractérisée en ce que** la phase huileuse de la préparation contient un ou plusieurs composants huileux qui sont choisis dans le groupe des composés adipate de diisopropyle, triglycéride d'acide caprylique/caprique (INCI Caprylic/Capric Triglycerides), palmitate d'isopropyle, diméthicone, cyclométhicone, octyldodécanol, cocoate d'éthylhexyle, myristate de myristyle, coco-glycérides hydrogénés (INCI Hydrogenated Coco-Glycerides), carbonate de dicaprylyle, hydroxystéaroylstéarate d'alkyle en C₁₈₋₃₈ (INCI C18-38 alkyl hydroxystearoyl stearate), adipate de di-n-butyle, dicaprylate/dicaprate de butylèneglycol (INCI Butylene glycol Dicaprylate/dicaprate), benzoate d'alkyle en C₁₂₋₁₅ (INCI C12-15 Alkyl Benzoate), benzoate de 2-phényléthyle (INCI Phenethyl Benzoate), tartrate de dialkyle en C₁₂₋₁₃ (INCI Di-C12-13 Alkyl Tartrate), cocoate de butylèneglycol (INCI : Butylene Glycol Cocoate), éther dicaprylylique, néopentanoate d'isodécyle, trimellitate de tridécyle, stéarate d'isopropyle, lactate d'alkyle en C₁₂₋₁₃ octanoate de 2-propylheptyle, sarcosinate d'isopropyllauryle.

11. Utilisation selon l'une quelconque des revendications précédentes, **caractérisée en ce que** la préparation contient un ou plusieurs filtres UV choisis dans le groupe des composés sels d'acide phénylène-1,4-bis-(2-benzimidazyl)-3,3'-5,5'-tétrasulfonique ; sels d'acide 2-phénylbenzimidazole-5-sulfonique ; 1,4-di(2-oxo-10-sulfo-3-bornylidèneméthyl)-benzène et ses sels ; sels d'acide 4-(2-oxo-3-bornylidèneméthyl)-benzènesulfonique ; sels d'acide 2-méthyl-5-(2-oxo-3-bornylidèneméthyl)-sulfonique ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-benzylidènecamphre ; salicylate d'éthylhexyle ; acide téréphtalidènedicamphosulfonique ; () 4-(diméthylamino)-benzoate de 2-éthylhexyle; 4-(diméthylamino)benzoate d'amyle ; 4-méthoxybenzalmalonate de di(2-éthylhexyle) ; 2-hydroxy-4-méthoxy-4'-méthylbenzophénone ; 2,2'-dihydroxy-4-méthoxybenzophénone ; 2-(4'-diéthylamino-2'-hydoxybenzoyl)-benzoate d'hexyle, 4-(tert-butyl)-4'-méthoxydibenzoylméthane ; salicylate d'homomenthyle ; 2-hydroxybenzoate de 2-éthylhexyle ; benzalmalonate de diméthicodiéthyle ; copolymère 3-(4-(2,2-bis éthoxycarbonylvinyl)-phénoxy)-propényl)-méthoxysiloxane / diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine (n° CAS 288254-16-0) ; 4,4',4"-(1,3,5-triazine-2,4,6-triyltriimino)-tris-benzoate de tris(2-éthylhexyle) (également : 2,4,6-tris-[anilino-(p-carbo-2'-éthyl-1'-hexyloxy)]-1,3,5-triazine (INCI : Ethylhexyl Triazone) ; 2,4-bis-{[4-(2-éthyl-hexyloxy)-2-hydroxy]-phényl}-6-(4-méthoxyphényl)-1,3,5-triazine ; 4-dicyanométhylène-2,6-diméthyl-1,4-dihydropyridine-N-(sels d'ester éthyloxysulfate),dioxyde de titane ; oxyde de zinc.

12. Utilisation selon l'une quelconque des revendications précédentes, **caractérisée en ce que** la préparation contient un ou plusieurs alcools choisis dans le groupe des composés éthanol, glycérol, éthylhexylglycérol, phénoxyéthanol, caprate de polyglyceryle-2, propylèneglycol, butylèneglycol, 2-méthylpropane-1,3-diol, 1,2-pentanediol, 1,2-hexanediol, 1,2-octanediol, 1,2-décanediol.

13. Préparation cosmétique selon l'une quelconque des revendications précédentes, **caractérisée en ce que** la préparation contient un ou plusieurs composés choisis dans le groupe des composés acide alpha-lipoïque, acide folique, phytoène, D-biotine, coenzyme Q10, alpha-glucosylrutine, carnitine, carnosine, isoflavonoïdes, flavonoïdes, créatine, créatinine, taurine, β-alanine, panthénol, polidocanol, acétate de tocophéryle, dihydroxyacétone, glycérylglucose, (2-hydroxyéthyl)urée, vitamine E ou acétate de vitamine E, acide hyaluronique et/ou ses sels sodiques, menthol, acide glycyrrhétinique et/ou licochalcone A.

14. Utilisation selon l'une quelconque des revendications précédentes, **caractérisée en ce que** la préparation contient un ou plusieurs polymères choisis dans le groupe des composés gomme xanthane, amidon de tapioca, hydroxyéthylcellulose, carbomère, acrylate/acrylate d'alkyle(C10-30), copolymère vinylpyrrolidone/hexadécène.
